# EUROPEAN PATENT APPLICATION

(11) **EP 3 539 645 A1**
(43) Date of publication of application: **18.09.2019**
(21) Application number: 18162343.0
(22) Date of filing: 16.03.2018
(51) Int. Cl.: B01D 67/00, B01D 69/02, B01D 69/14, B01D 71/82, C12N 11/08, C12N 11/14

(54) **FUNCTIONALISED MIXED MATRIX MEMBRANES AND METHOD OF PRODUCTION**

(71) Applicant: HurraH S.à r.l., 8281 Kehlen (LU)
(72) Inventor: LAINE, Carole Marie, B-6717 ATTERT (BE); DE MEESTER, Fabien, B-6900 Marche-en-Famenne (BE)
(74) Representative: Pronovem

(57) **Abstract**

A porous membrane having a porous matrix formed of a thermoplastic polymer material and inorganic filler particles embedded in the porous matrix, the inorganic filler particles having an accessible surface comprising nucleophilic groups bonded to the inorganic filler particles is functionalised by bringing the porous membrane in contact with an aqueous solution comprising a carboxylic acid or an anhydride thereof at a pH between 2 and 3.5 to obtain a carboxylic acid functionalised membrane.

## Description

### Field of the Invention

The present invention is related to porous membranes, in particular mixed matrix membranes comprising inorganic filler particles, wherein functional groups are bonded on a surface of the filler particles. The present invention is equally related to a method of functionalising such porous membranes.

### Background art

Functionalised porous silica filled membranes are known from US 4102746 to Goldberg, 25.07.1978 describing how to functionalise the membrane through covalent bonding of a bridging agent in the form of an organosilane (γ-aminopropyltriethoxysilane) directly to the filler particles to obtain 3-aminopropyl-silica, or alternatively through irreversible chemiadsorbing a bridging agent in the form of a macromolecular polyelectrolyte (polyethylenimine) to the filler particles followed by crosslinking an enzyme to the bridging agent by means of a bifunctional electrophilic crosslinker involving e.g. glutaraldehyde. A number of bio-catalysts were produced with the aforementioned chemistry and the following food grade enzymes: glucose oxidase (conversion of glucose to gluconic acid) and glucose isomerase (conversion of glucose to fructose).

GOLDBERG, B.S., Lactose hydrolysis of whey permeate using a continuous flow through immobilized enzyme system, North European Dairy Journal 1985, Vol.1, pages 5-11, describes a continuous immobilized enzyme reactor for hydrolyzing lactose or lactose in whey permeate into glucose and galactose. A fast, stable performance of the system is reported (90% conversion of the lactose after 1.5 minutes residence time for a 5% feed) at pH 4.5 with long service intervals (5000 h). Reported advantages are lower enzyme cost, no contaminants (allergens), no column (with drawbacks such as channeling, diffusion, handling), over the alternative processes of adding lactase into the milk or immobilizing it.

There is a growing interest in functionalization of silica particles with carboxylic acid (COOH) groups. These carboxylic acid functionalised silica particles find application in nanotechnology, food science and medicine. Different routes for functionalising silica particles with carboxylic acid groups are known, all involving a multistep procedure. AN, Y. et al. Preparation and self-assembly of carboxylic acid-functionalized silica, Journal of Colloid and Interface Science 2007, Vol. 311, pages 507-513 describes how to first amino-functionalize silica nanoparticles (SiO₂-NH₂) by a silanization with 3-aminopropyltriethoxysilane (APTES) and in a second step prepare carboxylic acid-functionalized silica nanoparticles (SiO₂-COOH) by a ring opening linker elongation reaction of the amine functions with succinic anhydride in N,N-dimethylformamide (DMF) at room temperature for 24 hours. POPOVA, M. D. et al. Carboxylic modified spherical mesoporous silicas as drug delivery carriers, International Journal of Pharmaceutics 2012, Vol. 436, pages 778-785 describes a two-steps process for obtaining carboxylic functionalized silica. A first spherical silica is modified with amino groups by reaction with 3-aminopropyltriethoxysilane (APTES) in ethanol or anhydrous toluene, washing with several portions of solvent and finally with water, and drying at room temperature, followed by azeotropic drying at 115°C. Thereafter, the amino modified silica is reacted with succinic anhydride in toluene at 60°C and treated for 24 h. MAHALINGAM, V. et al. Directed self-assembly of functionalized silica nanoparticles on molecular printboards through multivalent supramolecular interactions, Langmuir 2004, Vol. 20, pages 11756-11762 describes how to functionalise silica nanoparticles with β-cyclodextrin by first amino-functionalizing silica nanoparticles in a reaction with APTES in ethanol followed by carboxylation by reaction with glutaric anhydride in DMF. The carboxylic groups on the silica nanoparticles are then activated with *N'*-(3-dimethylaminopropyl)-*N*-ethylcarbodiimide hydrochloride and N-hydroxysuccinimide (NHS) and further with *β*-cyclodextrin.

Apart from being multistep procedures, the known routes for functionalising filler (silica) particles with carboxylic acid groups require the use of possibly toxic compounds and organic solvents, which may obstruct the use of the functionalised particles for food processing or medical/pharmaceutical applications. These processes are not environmentally friendly, laborious and costly.

### Summary of the invention

It is hence an object of the present invention to provide procedures for functionalising supports, in particular porous supports, such as filled or mixed matrix membranes, which are less laborious, environmentally friendly and/or economical.

It is an object of the present invention to provide procedures for functionalizing supports which involve food grade compounds, making the functionalised supports so obtained suitable for food processing or for medical and pharmaceutical applications.

In addition, it is an object of the present invention to provide functionalised supports as obtained or obtainable by the above procedures. Yet a further object is to provide activated functionalized supports as obtained or obtainable by the above procedures.

According to a first aspect of the invention, there is therefore provided a method of functionalising a porous membrane as set out in the appended claims. The membrane comprises a porous matrix formed of an advantageously thermoplastic polymer material, and inorganic filler particles, advantageously silica, embedded in the porous matrix. The inorganic filler particles in the porous matrix advantageously have an accessible surface comprising nucleophilic groups, e.g. hydroxyl groups, in particular silanol (SiOH) groups, bonded to the inorganic filler particles. According to the present aspect, the porous membrane is brought in contact, e.g. by immersion, with an aqueous solution comprising a carboxylic acid or an anhydride thereof. The aqueous solution is advantageously at a pH between 2 and 3.5, advantageously between 2.5 and 3.0. Such pH levels can be obtained when the carboxylic acid is added to the aqueous solution in an amount to obtain saturation of the aqueous solution with the acid. The aqueous solution therefore is advantageously saturated with the carboxylic acid and/or the anhydride of the carboxylic acid. The concentration of the carboxylic acid and/or of the anhydride thereof is advantageously equal to or higher than the saturation concentration. By so doing, a carboxylic acid functionalised membrane can be obtained. Methods according to the invention therefore provide a one-step procedure for acid-functionalisation of a porous support. As furthermore no ingredients other than water and an anhydride of a carboxylic acid are advantageously required, making the procedure economical and environmentally friendly.

The carboxylic acid is advantageously a polycarboxylic acid. The (poly)carboxylic acid is advantageously a food-grade (poly)carboxylic acid. Such acids advantageously have the formula HOOC-R¹-COOH, wherein R¹ is a bivalent group comprising between C₁-C₁₆ carbon atoms, preferably between C₂-C₆ carbon atoms. Preferred carboxylic acids are: succinic acid, glutaric acid and citric acid, of which succinic acid and its food grade derivatives (e.g. octenylsuccinic acid) are most preferred. The use of food-grade acids allows for obtaining a food-grade acid-functionalised support which can advantageously be used in the food and/or pharmaceutical or medical industry.

Methods according to present aspects may further comprise activation and/or derivatization reactions following the functionalization step as described above.

According to a second aspect of the invention, there are provided functionalised membranes as set out in the appended claims. The membrane comprises a porous matrix formed of an advantageously thermoplastic polymer material, and inorganic filler particles, advantageously silica, embedded in the porous matrix. The inorganic filler particles in the porous matrix have an accessible surface, e.g. accessible via interconnected pores. According to the present aspect, the accessible surface comprises carboxylic acid functional groups bonded to the inorganic filler particles. The carboxylic acid groups are advantageously covalently bonded to the inorganic filler particles. Membranes according present aspects may further comprise activated and/or derivatized carboxylic acid functional groups.

### Description of the drawings

Aspects of the invention will now be described in more detail with reference to the appended drawings, wherein same reference numerals illustrate same features and wherein:
Figure 1 represents the chemical structure following functionalisation of a silanol group with succinic anhydride according to an aspect of the invention;
Figure 2 represents the chemical structure following activation of the group of figure 1 with N-hydroxysuccinimide;
Figure 3 represents the chemical structure following derivatization of the activated ester group of figure 2 with an amine (i.e. amino acid, or peptide), alcohol (i.e. β-cyclodextrin), or any other nucleophilic group of interest;
Figure 4 represents derivatization of the activated ester group of figure 2 with a HABA-Avidin complex;
Figure 5 represents synthesis of a biotynilated protein for use with the HABA-avidin derivatized membrane of Figure 4 in Membrane Catalyst Bioreactors.

### Detailed description

### Membrane Supports

Membranes for use in methods according to the present invention comprise an advantageously porous matrix in which filler particles are embedded. Such membranes are referred to as mixed matrix membranes, or filled porous and possibly semipermeable membranes. A mixed matrix membrane refers to a membrane comprising a polymeric matrix in which an advantageously inorganic material is dispersed. The polymeric matrix is substantially made of a thermoplastic polymer material. A first class of suitable thermoplastic polymer materials are halogenated polymers such as but not limited to polyvinyl chloride (PVC) and polyvinylidene chloride (PVDC), polyolefins such as polyethylene and polypropylene, or acrylic polymer materials, such as a (meth)acrylate homo or copolymer, e.g. poly(methyl methacrylate) (PMMA). A second class of suitable thermoplastic polymer materials are polymers obtained from polymerization of one or more ethylenically unsaturated monomer(s) selected from the group consisting of C₁-C₂₀-alkyl (meth)acrylates, vinyl and allyl esters of carboxylic acids of up to 20 carbon atoms, vinyl ethers of C₁-C₈ alcohols, vinyl aromatics of up to 20 carbon atoms, ethylenically unsaturated nitriles, vinyl halides, C₁-C₁₀-hydroxyalkyl(meth)acrylates, (meth)acrylamide, (meth)acrylamide substituted on the nitrogen by C₁-C₄-alkyl, ethylenically unsaturated carboxylic acids, ethylenically unsaturated dicarboxylic acids, half-esters of ethylenically unsaturated dicarboxylic acids, anhydrides of ethylenically unsaturated dicarboxylic acids, non-aromatic hydrocarbons having at least two conjugated double bonds, C₁-C₈ alkenes and mixtures of these monomers. Specific examples of polymers of the second class are polyvinylacetate (PVAc), polypropylene and polyacrylate. The matrix can be made of a blend of any combination of the above polymers of the first class and/or second class, or can be made of, or comprise a copolymer of a halogenated monomer, olefin monomer, or acrylic monomer (e.g. in an amount of 75% mole or more) and a monomer as indicated in the second class above (e.g., up to 25% mole). Polyvinyl chloride is a preferred polymer for the matrix, advantageously obtained from unplasticised, vinyl chloride polymer resin.

The filler particles are advantageously mineral or inorganic particles, and they are advantageously porous, e.g. they feature a surface porosity. The filler particles advantageously comprise nucleophilic groups, such as but not limited to hydroxyl groups at an accessible location, e.g. on the surface and/or in accessible pores. Suitable materials for the filler particles are silicates, aluminates and (derivatized) zeolites. The filler particles may have a high specific surface area. Advantageously, the filler particles have a high amount of available/accessible nucleophilic groups, such as -OH groups, such as at least 1 µmol accessible -OH per square meter of particle surface area, advantageously at least 2 µmol/m², advantageously at least 4 µmol/m², advantageously about 5 µmol/m² and up to 8 µmol/m² or even more.

The filler particles advantageously comprise, or consist of silica (SiO₂) particles, in particular particles of amorphous silica or natural silica (diatomite), which are advantageously hydrophilic. Suitable silica particles are silica gel or precipitated silica (e.g., SIPERNAT® or ULTRASIL® or SYLOID® or PERKASIL®), and to a lesser extent colloidal and fumed silica particles. One important feature of the silica particles is the presence of silanol (SiOH) groups on the surface of the silica particles. Advantageously, the silica particles have a high surface density of silanol groups, such as at least 1 µmol/m² SiOH, advantageously at least 2 µmol/m², advantageously at least 4 µmol/m², advantageously about 5 µmol/m² and up to 8 µmol/m² of SiOH group.

The filler particles advantageously have mean particle size between 1 nm and 50 nm. The filler particles typically form aggregates in the matrix, with mean aggregate size possibly ranging between 100 nm and 50 µm, advantageously between 200 nm and 25 µm, advantageously between 500 nm and 20 µm. The aggregate size can be measured using a diffraction laser particle size analyzer, (as e.g. sold by CILAS, France), or based on image analysis.

The mixed matrix membranes as used in methods of the invention advantageously feature an interconnected porosity. Due to the aggregation of the filler particles, the porosity can have a bimodal pore size distribution. Finer pores (e.g. the voids between the filler particles in the aggregates, or the porosity of the filler particles) have typical pore size below 1 µm, advantageously 0.5 µm or less, advantageously 0.1 µm or less, advantageously 0.05 µm or less. Large pores, referred to as extraction pores, present in the porous matrix as a consequence of the manufacturing method (as described below), are typically greater than 1 µm in size, possibly at least 2 µm, possibly at least 3 µm. The volume porosity (i.e. the volume fraction of the voids) of the membranes is advantageously at least 50%, advantageously at least 60 %, advantageously at least 70%.

Advantageously, the amount of filler particles in the membrane is 35% by weight or more, advantageously 45% by weight or more, advantageously 50% by weight or more, advantageously 66% by weight or more. As the filler particles advantageously form aggregates and as the matrix is porous, the filler particles will typically comprise a surface of the particle which is accessible through the pores in the membrane. Advantageously, the mixed matrix membranes feature a specific surface area (BET) of at least 50 m²/g, advantageously at least 100 m²/g, advantageously at least 150 m²/g, and possibly 250 m²/g or more, e.g. up to 800 m²/g.

The membranes can have a dry weight of between 50 g/m² and 800 g/m², possibly between 100 g/m² and 500 g/m², with thickness possibly ranging between 0.2 mm and 2 mm, possibly between 0.5 mm and 1.5 mm. The dry density of the mixed matrix membranes advantageously falls between 1.0 g/cm³ and 2.0 g/cm³, advantageously between 1.5 g/cm³ and 1.8 g/cm³.

The filled porous membranes, in particular where silica is the filler particles advantageously show one or more of the following characteristics: non-compressible; resistant to sterilisation by steam; hydrophilic; and resistant to acids, alcohols and hydrocarbons. As an advantage, the filled porous membranes, where polyvinyl chloride is the thermoplastic polymer and silica is the filler is suitable for direct food contact since the free chloride (Cl⁻) from the polyvinyl chloride and any residual co-extrusion solvent, such as cyclohexanone are on the order of a few ppm (typically less than 50 ppm) and since the structure (matrix and silica) has a high chemical stability, e.g. it is highly resistant to strong acids and stable at low pH.

Such filled semipermeable membranes are known and used e.g. as separators in lead-acid batteries. They are manufactured starting from forming a dry powder blend from the filler particles and the thermoplastic polymer material, which is provided as a polymer resin, possibly in a ratio of polymer resin to filler particles between about 1:0.5 and 1:2.5. The resin can be formed by resin particles which are porous (e.g. paste PVC or emulsion polymerisation PVC). Alternatively, resin particles can have a hard, glossy beaded appearance (in other words suspension polymerisation PVC). An organic solvent, such as cyclohexanone, is added to the powder blend under agitation of the powder blend mixture, advantageously followed by adding a non-solvent, such as water, to prevent the solvent from solubilising the polymer resin completely. A free-flowing powder is so obtained which can be further processed into the porous membrane by e.g. extruding through a die and calendaring to the desired thickness and shape. Suitable shapes for use in aspects of the invention are flat, ribbed, and undulated sheets.

Further particulars of the membrane manufacturing steps and materials used for manufacturing the filled semipermeable membranes are described in US 3696061 to Selsor, J.Q. 03.10.1972, US 2005/0158630 to Lambert, U. 21.07.2005 and EP 2902094, 05.08.2015, the contents of which are fully incorporated herein by reference.

One advantage of the above described manufacturing route is that, since the polymer resin is not completely solubilised, but maintained as a powder, the surface of the filler particles, and in particular any surface pores, are not coated/filled by the polymer, and therefore remain accessible. The above membranes therefore feature an increased/enhanced accessibility of nucleophilic groups present on the particle surface within the membrane matrix.

### Acid functionalisation

In an aspect of the invention, porous membranes, such as those obtained by the above methods, are acid-functionalised, in particular with a carboxylic acid group. Advantageously, the functionalisation with the carboxylic acid group is obtained by carboxylation of the nucleophilic (e.g., -OH) groups present on the surface of the filler particles. Advantageously, the carboxylic acid functionalisation leads to a carboxylic acid group which is covalently bonded to the filler particles.

To functionalise the membrane, use is made of an organic acid anhydride, advantageously an anhydride of a carboxylic acid, more particularly an anhydride of a polycarboxylic acid. The polycarboxylic acid can be an aliphatic or cycloaliphatic polycarboxylic acid. Alternatively, the polycarboxylic acid can be an aromatic polycarboxylic acid such as ortho phthalic acid, 3-fluoro orthophthalic acid, 4-fluoro orthophthalic acid, 2,3-pyridinedicarboxylic acid and pyrazine dicarboxylic acid. Anhydrides of aliphatic polycarboxylic acids are preferred. The aliphatic polycarboxylic acids can be linear or cyclic, branched or unbranched, saturated or unsaturated. The polycarboxylic acid can be a dicarboxylic acid, advantageously an α,ω-dicarboxyiic acid. The polycarboxylic acid can have the formula HOOC-R¹-COOH, wherein R¹ is a bivalent (organic) group, advantageously a bivalent hydrocarbon group, and which can comprise suitable substituents, such as hydroxyl (-OH), (cyclo)alkyl, allyl, carboxyl, carboxymethyl (-CH₂-COOH) and acetyl (-CO-CH₃) substituents. Advantageously, R¹ comprises between 1 and 16 carbon atoms, advantageously at least 2 carbon atoms, and advantageously 10 carbon atoms or less, advantageously 8 or less. Advantageously, R¹ is a saturated or unsaturated aliphatic group, and which can comprise or consist of linear and branched aliphatic groups. R¹ may comprise or consist of a cycloaliphatic group. Advantageously, R¹ is a bivalent C₁-C₁₆ aliphatic group, advantageously a bivalent C₂-C₁₆ aliphatic group, advantageously a bivalent C₂-C₁₀ aliphatic group. Alternatively, R¹ may itself comprise an acid anhydride functional group (e.g., HOOC-R'-CO-O-CO-R"-COOH with R' and R" being similar or different bivalent organic groups). R¹ optionally comprises one or more heteroatoms selected from the group consisting of oxygen, nitrogen, phosphorus, halogen and sulphur.

Suitable carboxylic acid anhydrides for use in methods according to the present aspect of the invention are maleic anhydride, succinic anhydride, glutaric anhydride and derivatives thereof, comprising citric anhydride, methylsuccinic anhydride, dimethylsuccinic anhydride, cyclopentanedicarboxylic anhydride, cyclohexanedicarboxylic anhydride, phenylsuccinic anhydride, octadecenylsuccinic anhydride, dodecenylsuccinic anhydride, nonenylsuccinic anhydride, (2-octen-1-yl)succinic anhydride, (2-dodecen-1-yl)succinic anhydride, (2-nonen-1-yl)succinic anhydride, 2-(1-tetradecenyl)succinic anhydride, 3-[(1E)-1 -decenyl]dihydro-2,5-furandione, methyl-5-norbornene-2,3-dicarboxylic anhydride, bicyclo[2.2.1]hept-5-ene-2,3-dicarboxylic anhydride, endo-bicyclo[2.2.2]oct-5-ene-2,3-dicarboxylic anhydride, cis-5-norbornene-exo-2,3-dicarboxylic anhydride, exo-3,6-epoxy-1,2,3,6-tetrahydrophthalic anhydride, cantharidin, norcantharidin, acetoxysuccinic anhydride, formamidosuccinic anhydride, diacetyl-L-tartaric anhydride, aspartic anhydride, triphenylphosphoranylidenesuccinic anhydride, S-acetylmercaptosuccinic anhydride, trifluoroacetamidosuccinic anhydride, and tetrafluorosuccinic anhydride.

According to an aspect of the invention, the anhydride as defined above is added to a water bath advantageously at room temperature, or slightly elevated temperature, e.g. temperatures between 10°C and 60°C. The anhydride will convert to its corresponding acid in the water bath. The acid will act as reactant-catalyst and react with the silanol-groups on the filler particles. It has been observed that the carboxylic acid functionalisation is stable at a pH equal to or smaller than 3.5, advantageously equal to or smaller than 3. The pH is advantageously at least 2, advantageously at least 2.5. The indicated pH levels can be obtained when saturating the water bath with the (acid of the) anhydride. A saturation level can refer to a level in which the acid of the anhydride is in excess, such that a precipitate and/or sediment (of the anhydride and/or the corresponding acid) is present in the water bath. By way of example, it was shown that a saturated level of succinic anhydride (SAH, i.e. the anhydride of succinic acid) in water at equilibrium and room temperature is 6.2% w:w or 0.62 M (where the molecular weight of SAH = 100 Da). By way of example, the pH of a saturated aqueous solution of SAH at equilibrium and room temperature is about 2.7.

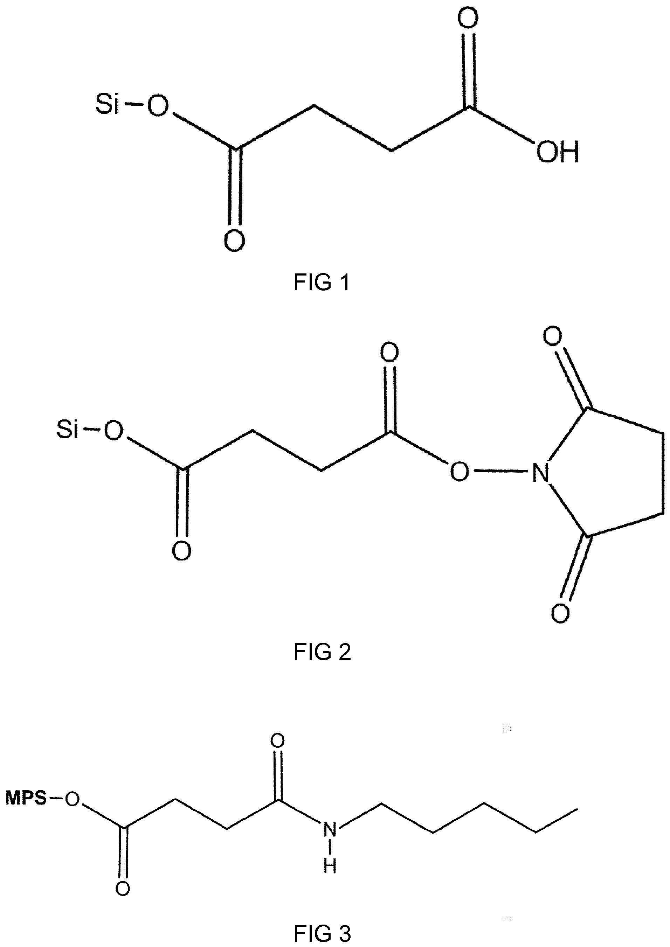

The membrane is immersed/soaked in the above indicated saturated (reactant) water bath. Optimal reactant concentration (saturation) is maintained by keeping the anhydride in suspension in the water bath, e.g. by stirring with a mechanical or magnetic stirrer. It was surprisingly found that the contact of a silica filled membrane with the saturated water bath caused carboxylic acid groups from the anhydride to bond/graft to the silanol groups on the surface of the embedded silica particles. Covalent bonds are advantageously formed between the silica and the carboxylic acid groups. These bonds can be according to the formula Si-O-CO-R¹-COOH, wherein R¹ represents a bivalent group as indicated above. By way of example, in case of succinic anhydride, the covalent bonds are according to the formula Si-O-CO-(CH₂)₂-COOH. Figure 1 shows the functional carboxylic acid group in case succinic anhydride is used.

As filler particles in the core of the membrane are accessible through the pore network of the membrane, the accessible filler particle surface in the internal of the membrane is advantageously functionalised with the carboxylic acid groups as well. Advantageously, the functionalised membrane comprises at least 0.1 mmol available carboxylic acid groups per gram of membrane, advantageously at least 0.2 mmol COOH/g membrane.

Even more surprising was that the above process of carboxylic acid functionalisation works at room temperature, which is generally referred to as a temperature between 15°C and 35°C. An immediate reaction (e.g., about 1 minute) was observed in the case of SAH at room temperature. The water bath is therefore advantageously kept at room temperature, and no higher temperatures are required, so that methods according to the invention are more economical and advantageously faster as well.

The process of carboxylic acid functionalisation is advantageously carried out at atmospheric pressure. In particular, the saturated water bath is advantageously maintained at atmospheric pressure.

The functionalised membrane, following immersion/soaking in the saturated water bath, can further be processed according to standard procedures. Advantageously, the membrane can be rinsed off with advantageously acidic water solution, advantageously water having a pH of 4 or less, advantageously a pH of 3.5 or less, e.g. a pH of 2.7. The pH can be adjusted with HCI. To this end, the membrane is immersed in a second water bath at acidic pH and possibly fed with running water to rinse off any excess anhydride absorbed in/on the functionalised membrane. Following rinsing, the functionalised membrane is advantageously dried, e.g. in a standard convection mode or infra-red or, microwave oven, advantageously at a temperature between 50°C and 75°C, until an acceptable level of humidity in the membrane is obtained.

It will be convenient to note that the carboxylic acid functionalisation can be performed as a continuous process or in batch mode. In the continuous process, a continuous sheet of the membrane is unwound from a roll and immersed in the water bath saturated with the anhydride, followed by immersion in a suitably acidic second water bath for rinsing. In the first, anhydride-saturated water bath, a continuous supply of the anhydride will generally be required in order to maintain the saturation level, i.e. to compensate for the anhydride which reacts with the membrane, and the anhydride possibly carried along with the membrane (and which will be rinsed off in the second water bath). In the batch mode, the membrane, pre-cut to final dimensions, may be immersed/soaked in a cartridge or other container filled with the first water bath for a predetermined reaction time. The membrane can be removed from the cartridge and immersed in a second container filled with the second water bath for rinsing.

In an alternative aspect, the inorganic filler particles are functionalised prior to manufacturing the membrane. Such a functionalisation can proceed through same reaction conditions as described above, e.g. by immersion/soaking in the reactant water bath, followed by rinsing and drying. The functionalised filler particles so obtained are then used for manufacturing the mixed matrix membranes as described above.

### Food-grade functionalisation

In a particularly advantageous aspect of the present invention, the acid functionalisation as described above allows for obtaining food grade functionalised membranes. An anhydride of a food grade, dietary or naturally occurring carboxylic acid is used to this end. Suitable food grade type carboxylic acids advantageously have the formula HOOC-R¹-COOH with R¹ a bivalent (organic) group as described above and which advantageously comprises between C₂-C₆ carbon atoms. Non limiting examples of food grade carboxylic acids are maleic acid, succinic acid, glutaric acid and citric acid. Experiments conducted by the inventors, described below, have shown that the functionalisation process works surprisingly well with anhydrides of food grade carboxylic acids, in particular with succinic anhydride. Since the functionalisation is carried out in water, advantageously without any other additive, the food compatibility of the functionalisation follows self-evidently provided the support (membrane) is food grade, which is the case particularly for the silica-filled PVC membrane described above, and which may be the case for other mixed matrix membranes, in particular those comprising an (food grade) inorganic filler and a (food grade) thermoplastic, e.g. polyolefinic, matrix.

The simplicity and lack of particular hazards in the above functionalisation process allows for carrying it out either in factory or advantageously even at the end user. In the latter case, the end user may be supplied with the pre-functionalised membrane, and the carboxylic acid anhydride either in powder form or in solution.

Since the functionalisation involves just an aqueous solution of a carboxylic acid anhydride, and may not present any particular hazards, e.g. where food grade type carboxylic acids are used, the functionalized membranes so obtained are particularly suitable for further use for food and medical/pharmaceutical applications. The carboxylic acid groups bonded on the surface of the filler particles in the functionalised membrane are advantageously available for ion exchange and/or further activation and/or derivatization as described below.

### Functionalised membranes

The functionalised membranes as obtained through procedures as described above advantageously feature an elevated concentration of carboxylic acid functional groups grafted/bonded on the filler particle surface. Advantageously, the grafting level amounts to at least 0.05 mmol COOH groups/g of membrane, advantageously at least 0.3 mmol COOH/g of membrane and possibly up to 2 mmol COOH/g of membrane. Grafting levels can be obtained by titration (e.g., see the examples below).

### Activation reactions

In a further aspect of the invention, the carboxylic acid functionalised membrane is activated, advantageously by activating the carboxylic acid functional group grafted/bonded on the (filler particles of) the membrane. An activation may make the membrane suitable for intended applications. One possible type of activation is an esterification of the available carboxylic acid group to form an activated acid (ester) which is bonded to the filler particles of the membrane. An activating reagent for carboxylic acid can be used for this purpose to obtain a carboxylate ester.

A suitable activating reagent for esterification of the carboxylic acid group can be an N-hydroxyalkylimide, which can have the formula: R²-CO-NOH-CO-R³, wherein R² and R³ are independently selected from the group consisting of C₁-C₆ alkyl, C₁-C₆ alkenyl, C₁-C₆ alkynyl, C₁-C₆ acyl, optionally comprising an ethylenically unsaturated double bond, and (meth)acryloyl C₁-C₆ moiety, the C₁-C₆ part of said moieties being normal chained, branched or cyclised and being optionally substituted by one or more moieties selected from the group consisting of C₁ - C₄ hydrocarbon, aryl and aralkyl and optionally comprising one or more heteroatoms selected from the group consisting of oxygen, nitrogen and sulphur and optionally comprising one or more carbonyl moieties. Alternatively, R² and R³ are forming a ring structure wherein CO-NOH-CO is incorporated in the ring and wherein the ring is optionally substituted by one or more moieties selected from the group consisting of C₁ - C₄ hydrocarbon, sulfonate (SO₃⁻X⁺, wherein X⁺ is selected from the group of H⁺, Na⁺, K⁺, Li+ and NH₄⁺), halogen, aryl and aralkyl and optionally comprises one or more heteroatoms selected from the group consisting of oxygen, nitrogen and sulphur and optionally comprises one or more carbonyl moieties. A preferred N-hydroxyalkylimide is N-hydroxysuccinimide (NHS).

The activation reaction proceeds with immersing/soaking the functionalised membrane in a solution of the N-hydroxyalkylimide, optionally in the presence of a coupling reagent. This will substitute the hydrogen atom of the carboxylic acid group on the functionalised membrane with a N-oxyalkylimide radical.

A possible type of esterification involves immersing/soaking the functionalised membrane in an anhydrous solution of NHS to form an NHS-activated acid on the available carboxylic acid group. A known type of NHS-activation involves using dicyclohexylcarbodiimide (DCC) or ethyl(dimethylaminopropyl) carbodiimide (EDC) as coupling reagent in the anhydrous solution of NHS.

Advantageously, the activated membrane so obtained comprises an activated acid bonded to the filler particles of the membrane according to the formula: Si-O-CO-R¹-CO-OR⁴, where R¹ is as indicated above, and OR⁴ represents a deprotonated activating reagent for carboxylic acid (e.g. deprotonated NHS), with R⁴ advantageously being a heterocyclic compound comprising nitrogen, advantageously having the formula R²-CO-N-CO-R³ with R² and R³ as indicated above. Advantageously, the -OR⁴ group is a leaving group in any derivatization reaction, e.g. for forming an amide or peptide bond as described below. Figure 2 shows the activated ester group in case NHS is used as activating reagent.

### Derivatization reactions

In yet a further aspect of the invention, the activated membrane is derivatized to make the membrane suitable for further intended applications, in particular in the field of biotechnology. Advantageously, the derivatization comprises forming an amide or a peptide bond on the activated carboxylic acid group, e.g. the carboxylate ester group obtained in the activation step described above. Any suitable amine, amino acid, peptide or functional protein can be used to this end. The derivatization can comprise an amidation of the activated carboxylic acid group to form a carboxamide group bonded to the filler particles, advantageously by reacting the activated membrane with a suitable amine, or amino-acid. Derivatized membranes can be used in enzymatic binding processes according to known methods.

The derivatized membrane so obtained advantageously comprises amide or ester groups bonded to the filler particles. One example of such a group bonded to a silica particle is according to the formula Si-O-CO-R¹-CONR⁵R⁶, where R¹ is as indicated above, R⁵ advantageously is hydrogen and R⁶ advantageously represents an organic group, such as an amino acid, a residue of an amino acid, a peptide, a polypeptide, a protein, an enzyme, an alcohol, or any nucleophile-bearing substituents. Figure 3 shows the carboxamide bond.

Advantageously, the derivatization comprises reacting the activated membrane with a (poly)peptide, e.g. a functional protein. One example of a functional protein is avidin. The peptide is made to react with the activated ester group. One example is to react the peptide with the -OR⁴ group on the available ester group bonded to the filler particles of the activated membrane. By so doing, the OR⁴ group is advantageously substituted, e.g. with a deprotonated peptide.

One suitable derivatization reaction involves the use of an avidin-biotin system. To this end, the derivatization advantageously comprises reacting the activated membrane with avidin. This allows for applications in which the derivatized membrane (with e.g. avidin end groups bonded to the filler particles) binds biotinylated compounds to the avidin end groups. Such biotinylated compounds may e.g. be ligands (e.g. β-cyciodextrin, metal chelates), antibodies (e.g. IgG, IgY), hormones, enzymes (e.g. phospholipase, glucose oxidase), or the like, and which are suitable for use in the avidin-biotin system.

Through any of the above aspects according to the invention, the filled porous membrane becomes suitable for a vast number of applications, such as:
- Egg & Milk processing, such as de-sugaring (lactase, glucose oxidase), emulsifying (lipases, phospholipases), hydrolyzing (proteases);
- Vegetable oil degumming (phospholipases), hydrolyzing (lipases)
- Fruit juices process throughput & product quality (pectinases, cellulases, amylases)
- Brewery process throughput & product quality (proteases);
- Laundry detergents / bleaching (oxidases) & defuzzing (cellulases); and
- Bioethanol extraction / fermentation.

The filled porous membrane, which has been functionalized, activated and/or derivatized according to the above methods is advantageously used in membrane reactors, such as membrane bioreactors for enzymatic processes.

An advantage of aspects according to the present invention is that the filler particles on which the active groups are bonded are immobilised in the porous matrix of the membrane, while these remain accessible to any liquid or gaseous substance which is made to pass through the membrane. With respect to packed columns, functionalised or activated membranes according to aspects of the invention have the further advantage to make post-processing more easy, as the membranes are more easy in handling with respect to loose particles of the column.

### Examples

### Example 1: Functionalisation by carboxylation with succinic anhydride

A 50 wt% silica filled porous polyvinyl chloride (PVC) membrane of 10 m long x 50 cm wide was unwound from a roll and passed at a constant speed of 50cm/min through a first 25L bath of water maintained at saturation of SAH (6.2% w:w or 0.62 M) at room temperature (pH∼2.5) to obtain a residence time of the membrane in the water bath of about 1 min. From the first bath, the membrane was subsequently passed in-line through a second similarly sized bath constantly fed with running water and maintained at pH 2.7 by addition of HCI to rinse off all excess SAH absorbed in/on the membrane prior to entering an air-circulating oven at 65°C for continuous in-line drying until a relative humidity less than 0.1% was obtained.

The water absorption capacity (WAC) of the pre-functionalised silica-filled PVC membrane (weight of 300g/m²) was determined to be 585 g/m² and the theoretical concentration of *per se* available OH groups to be 0.5 mmol/g silica. At the indicated WAC value, the pre-functionalised membrane absorbs 36.3 g (0.36 mol) SAH/m² as calculated from the SAH concentration. Acid-base titration of the SAH-treated membrane measured a grafting level of 0.10 mol COOH/m². The reactant consumption therefore averages 0.1/0.36-27.5%, all excess being washed off in the second bath. Hence, a binding capacity of about 10 µmol/cm², i.e. 0.25 mmol/g membrane in accordance with the theoretical OH groups concentration of the pre-functionalised membrane was obtained. Considering a standard protein of 50kDa molecular weight, the obtained level of functionalisation would translate into a 1:1 (mol:mol) theoretical binding capacity of ±10 µmol/cm² x 50,000 g/mol = 500 mg/cm² of functionalised membrane.

### Example 2: Activation by esterification with N-hydroxysuccinimide

The dried functionalised membrane obtained from example 1 was passed in a third 25L bath of 97% denaturated ethanol saturated with a 5% w:v of a mixture of 98% N-hydroxysuccinimide (Sigma-Aldrich® No. 130672) and 99% dicyclohexylcarbodiimide (Merck® No. 802954) preliminary co-blended in a 1:2 w:w ratio, at the same speed as in example 1. An immediate production of dicyclohexylurea (DCU) was observed at the surface of the membrane and the reaction was slightly exothermic. From the third bath, the membrane was subsequently passed in-line through a fourth similarly sized bath filled with 97% denatured ethanol to rinse off / sediment all produced DCU absorbed in/on the membrane prior to entering an air-circulating oven at 65°C for continuous in-line drying.

The sedimented DCU in the fourth bath was collected, dried and weighed for yield calculation. The produced DCU is considered to reflect the number of Si-OH groups which have been grafted with carboxylic acid groups in example 1. Based on the theoretically expected Si-OH groups available of about 0.25mmol/g (as determined in example 1 above), DCU yield calculation estimates a grafting level of about 100%±10%.

### Example 3: Derivatization by amidation with avidin

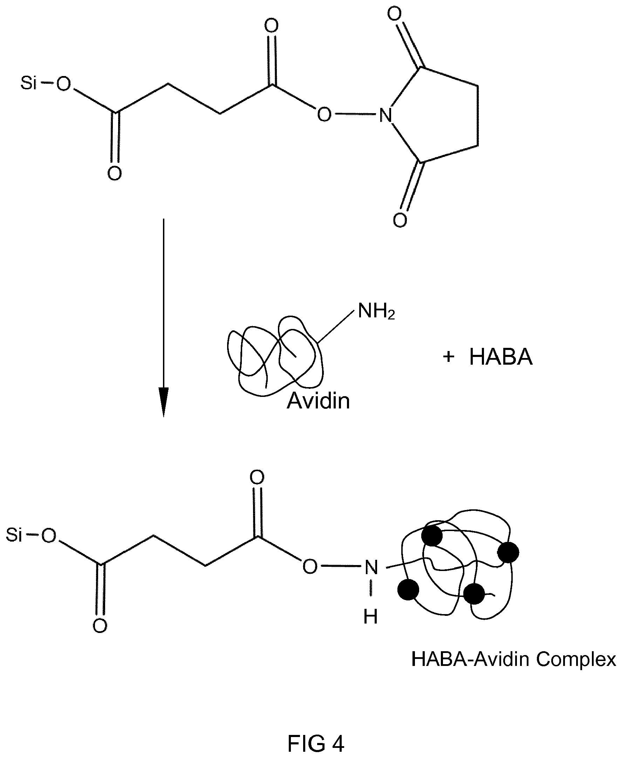

The dried activated membrane obtained in example 2 was treated in batch or passed at a constant speed of 50cm/min through a fifth 25L 50mM sodium phosphate (NaPi) buffer bath (pH=7.0) containing Avidin (AVI, eProtein Avidin, Batch AVI-25, min 12.0 U/mg) at a concentration of (1 to 50 g/10 L (batch)) and 2-(4-hydroxyphenylazo)benzoic acid (HABA, Sigma-Aldrich® No. H5126), which is used as indicator (ε⁵⁰⁰: 34,000M⁻¹cm⁻¹), at a concentration of (0.025g to 0.75 g/10L (batch)), to obtain a residence time of about 1 min to 24 hours (batch) in the bath at room temperature. From the fifth bath, the membrane was subsequently passed in-line through a sixth similarly sized bath filled with water to rinse off all excess reactants in/on the membrane prior to entering an air-circulating oven at 70°C for continuous in-line drying. A faint orange/red level of coloration of the avidin-HABA complex (absorbing at 500nm) was obtained for the membrane prior to drying. Synthesis of the HABA-Avidin derivatized membrane is schematically represented in Fig. 4.

### Example 4: Derivatization by amidation with avidin (batch test)

Two strips of 100 cm length were cut from the dried activated membrane obtained in example 2 and left to soak in a 50mM NaPi buffer (pH=7) containing AVI (5g/L or 75µM batch) for 24 h at 20°C. The strips were then soaked back in 50mM NaHCO3 (pH=7) containing HABA (75mg/L or 300µM bench DMF - molecular weight: 242 Da), while mixing by hand for 15 min and left to incubate for 24 h at 20°C. The strips turned orange during mixing in the HABA bath, then intensely red through formation of the avidin-HABA complex. The strips were subsequently left to dry in an air-circulating oven at 65°C.

### Example 5: Derivatization for membrane catalyst bioreactor

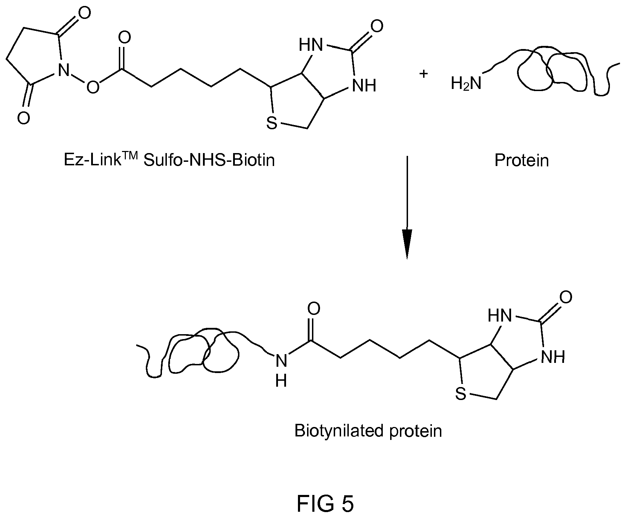

An enzyme comprising primary amino groups in water (10 g/l) was dialyzed against a 50mM sodium phosphate buffer (pH=7.5) and then mixed in a 2:1 molar ratio with Ez-Link™ Sulfo-NHS-Biotin (a long chain water soluble biotinylation agent from ThermoFisher Scientific) and left incubated overnight at 4°C to form an amide bond (see synthesis scheme of Fig. 5). The biotinylated enzyme was dialyzed against a 50 mM sodium phosphate buffer and then combined with the avidin-HABA derivatized membrane obtained in example 3, (absorbing at 500 nm), until saturation (4 biotin conjugated enzyme per avidin molecule). The membrane saturated with derivatized avidin-biotin-enzyme groups (AVI-B-Ez) is ready for being used as Membrane Catalyst Bioreactor.

### Example 6: Functionalisation by carboxylation with other anhydrides

The same procedure of Example 1 was repeated twice, in which succinic anhydride was replaced by citric acid anhydride in the first case and by glutaric acid anhydride in the second case. DCC/NHS titration with DCU yield calculation following the procedure of example 2 above resulted in a grafting level of 85% for citric acid anhydride and 88% for glutaric acid anhydride.

## Claims

1. Method of functionalising a porous membrane, wherein the porous membrane comprises:
a porous matrix formed of a thermoplastic polymer material; and
inorganic filler particles embedded in the porous matrix, the inorganic filler particles having an accessible surface comprising nucleophilic groups bonded to the inorganic filler particles,
**characterised in that** the method comprises bringing the porous membrane in contact with an aqueous solution comprising a carboxylic acid and/or an anhydride thereof at a pH between 2 and 3.5, preferably between 2.5 and 3.0, to obtain a carboxylic acid functionalised membrane.

2. Method of claim 1, wherein the nucleophilic groups are silanol (SiOH) groups.

3. Method of claim 1 or 2, wherein the inorganic filler particles are silica.

4. Method of any one of the preceding claims, wherein the aqueous solution is at room temperature and substantially at atmospheric pressure during contact with the porous membrane.

5. Method of any one of the preceding claims, wherein the carboxylic acid is a polycarboxylic acid.

6. Method of claim 5, wherein the polycarboxylic acid is according to the formula HOOC-R¹-COOH, wherein R¹ is a bivalent group comprising between C₁-C₁₆ carbon atoms, preferably between C₂-C₆ carbon atoms.

7. Method of any one of the preceding claims, wherein the carboxylic acid is a food grade type acid.

8. Method of any one of the preceding claims, wherein the carboxylic acid is one of a group consisting of succinic acid, glutaric acid, citric acid and derivatives thereof.

9. Method of any one of the preceding claims, comprising esterifying a carboxylic acid group of the carboxylic acid functionalised membrane to obtain a carboxylate ester activated membrane.

10. Functionalised membrane, comprising a porous matrix formed of a thermoplastic polymer material and inorganic filler particles embedded in the porous matrix, the inorganic filler particles having an accessible surface,
**characterised in that** the accessible surface comprises carboxylic acid groups bonded to the inorganic filler particles.

11. Membrane of claim 10, wherein the inorganic filler particles are silica.

12. Membrane of claim 10 or 11, wherein the carboxylic acid groups form covalent bonds with the inorganic filler particles.

13. Membrane of claim 12, wherein the bonds are according to one formula of a group consisting of Si-O-CO-COOH and Si-O-CO-R¹-COOH, wherein R¹ is a bivalent group comprising between 1 and 16 carbon atoms, preferably between 2 and 6 carbon atoms.

14. Activated membrane, comprising a porous matrix formed of a thermoplastic polymer material and inorganic filler particles embedded in the porous matrix, the inorganic filler particles having an accessible surface,
**characterised in that** the accessible surface comprises activated carboxylic acid groups, in particular carboxylic acid N-hydroxysuccinimide ester groups, bonded to the inorganic filler particles.

15. Membrane of claim 14, wherein the inorganic filler particles are silica, and comprising the carboxylic acid N-hydroxysuccinimide ester groups forming bonds with the silica particles according to the formula Si-O-CO-R¹-COOR⁴, wherein COOR⁴ represents the carboxylic acid N-hydroxysuccinimide ester group and R¹ represents a bivalent group comprising between 1 and 16 carbon atoms, preferably between 2 and 6 carbon atoms.
